# NEUE EUROPÄISCHE PATENTSCHRIFT

(11) **EP 2 979 667 B2**
(45) Veröffentlichungstag und Bekanntmachung des Hinweises auf die Entscheidung über den Einspruch: **28.10.2020**
(45) Hinweis auf die Patenterteilung: 09.08.2017
(21) Anmeldenummer: 15172372.3
(22) Anmeldetag: 16.06.2015
(51) Int. Cl.: A61F 2/966, A61F 2/24, A61F 2/95, A61F 2/962

(54) **EINFÜHRVORRICHTUNG FÜR EIN MEDIZINISCHES IMPLANTAT IN EINEN MENSCHLICHEN UND/ODER TIERISCHEN KÖRPER**
INSERTION DEVICE FOR INSERTION OF A MEDICAL IMPLANT INTO A HUMAN AND/OR ANIMAL BODY
DISPOSITIF D'INTRODUCTION D'UN IMPLANT MÉDICAL DANS UN CORPS HUMAIN ET/OU ANIMAL

(30) Priorität: 30.07.2014 US 201462030623 P
(43) Veröffentlichungstag der Anmeldung: 03.02.2016
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- EP-A1- 1 004 327
- EP-A2- 0 747 022
- EP-A2- 0 850 653
- WO-A1-2011/035327
- WO-A1-2011/130006
- WO-A1-2011/133368
- WO-A2-2010/022138
- US-A1- 2003 199 852
- US-A1- 2007 005 131
- US-A1- 2009 287 292
- US-A1- 2010 262 227
- US-A1- 2012 101 561
- US-B2- 6 699 273
- US-B2- 8 690 905

## Beschreibung

Die Erfindung betrifft eine Einführvorrichtung, insbesondere einen Katheter, zum Einführen eines medizinischen Implantats in einen menschlichen und/oder tierischen Körper gemäß Ansprüchen 1, 9 und 17.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents. Auf dem Feld der Herzimplantate sind beispielsweise Klappenimplantate, wie etwa Aortenklappenimplantate, bekannt, welche die Funktion der natürlichen Aortenklappe übernehmen.

Implantate werden vor einem Einführen in den Körper mit Kathetern verbunden und müssen so befestigt sein, dass sie am Einsatzort komplikationslos vom Katheter genau platziert und definiert freigegeben werden können. Ein häufiges Problem ist hierbei, das Implantat an einer definierten Position zu platzieren. Wird das Implantat in einer Fehlstellung platziert, kann dies zu einem Versagen des Implantats führen. Dies tritt beispielsweise oft bei Kalzifizierung, d.h. die Ablagerung von Kalziumsalzen, insbesondere Kalziumphosphat (Hydroxylapatit) an den Strukturen des Herzens und insbesondere bei stark asymmetrisch kalzifizierte Aortenstenose auf. Daher ist häufig vorgesehen, das freigesetzte Implantat wieder ganz oder teilweise in die Einführvorrichtung zurückzuziehen und entweder zu entfernen oder an anderer Stelle wieder freizusetzen. Bei einer Katheterlänge von einem Meter und mehr, stellt dies für die Einführvorrichtung eine mechanische Belastung dar, zumal die Einführvorrichtung entlang ihrer Längserstreckung Krümmungen und Biegungen erlauben muss.

US2009/0287292 A1 und US 2012/101561 A1 beschreiben eine Einführvorrichtung.

Der Erfindung liegt die Aufgabe zugrunde, eine Einführvorrichtung zu schaffen, die eine sichere Freisetzung und gegebenenfalls ein sicheres Zurückholen des Implantats in die Einführvorrichtung erlaubt.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird eine Einführvorrichtung zum Einführen eines medizinischen Implantats in einen menschlichen und/oder tierischen Körper vorgeschlagen, welche wenigstens einen Außenschaft aufweist mit einem proximalen Ende und einem diesem gegenüberliegenden distalen Ende, und welches ein mit dem Außenschaft verbundenes Aufnahmeelement für das Implantat aufweist, wobei der Außenschaft und das Aufnahmeelement an einem Übergang zwischen Außenschaft und Aufnahmeelement von einer lokal platzierten Hülle umgeben sind, welche mechanische Zug- und/oder Druckspannungen aufnimmt.

Vorteilhaft stabilisiert die Hülle den Bereich der Einführvorrichtung, welcher insbesondere im Einsatz starken Zug- und/oder Druckspannungen ausgesetzt ist. Der Außenschaft ist im bestimmungsgemäßen Einsatz die äußere Begrenzung der Einführvorrichtung und kann einen oder mehrere Innenschäfte umgeben. Der Außenschaft der Einführvorrichtung ist zwangsläufig flexibel ausgebildet, um das Implantat an seinen Bestimmungsort im Körper zu bringen. Die Katheterbelastungen im gebogenen Zustand erzeugen mechanische Spannungen. Werden diese Spannung gedämpft, kann eine Deformation bzw. ein Knick des Außenschafts vermieden werden.

Die erfindungsgemäße Einführvorrichtung erlaubt eine sichere Freigabe des Implantats aus der Einführvorrichtung. Typischerweise ist das Aufnahmeelement, eine Kapsel oder dergleichen, in der das Implantat an seinen Bestimmungsort geführt wird, am distalen Ende der Einführvorrichtung angeordnet. Einführvorrichtungen wie z.B. Katheter weisen Längen von mehr als einem Meter auf, so dass am distalen Ende ein Knickrisiko besteht. Ein Knick im Schaft, insbesondere im Außenschaft der Einführvorrichtung, kann die Freisetzung des Implantats erschweren oder sogar unmöglich machen. Ebenso kann ein Zurückführen des Implantats in das Aufnahmeelement erschwert oder unmöglich gemacht werden.

Die lokal platzierte Hülle am distalen Außenschaft erlaubt eine sichere Freigabe des Implantats aus dem Aufnahmeelement und, bei Bedarf, ein sicheres Zurückführen des Implantats in das Aufnahmeelement.

Besonders günstig ist die erfindungsgemäße Einführvorrichtung bei der Freisetzung von selbstexpandierenden Stentsystemen, zum Rückziehen des Schutzes von ballonexpandierenden Stents oder zum Rückziehen des Schutzes von beschichteten Ballonen. Das Design ist einfach zu realisieren und preiswert.

Gemäß einer günstigen Ausgestaltung kann die Hülle wenigstens ein proximales Ende des Aufnahmeelements überdecken. Typischerweise ist das Aufnahmeelement mit dem Außenschaft fest verbunden, z.B. verklebt. Dieser Bereich der Einführvorrichtung ist besonders knickgefährdet.

Gemäß einer günstigen Ausgestaltung kann die Hülle aus einem Material gebildet sein, das bezogen auf seinen Innendurchmesser von einem geweiteten Zustand gezielt in einen geschrumpften Zustand kontrahierbar ist. Insbesondere kann die Hülle aus einem Thermoplasten gebildet sein, der sich bei Einwirken von Wärme zusammenzieht. Vorteilhaft ist der Einsatz von PEEK (Polyetheretherketon). Denkbar ist auch ein Material, das sich auch ohne Wärmeeinwirkung zusammenzieht, wie etwa so genannte Kaltschrumpfschläuche aus EPDM (ethylene-propylene-diene-monomer, Ethylen-Propylen-Dien-Kautschuk) oder Silikon.

Gemäß einer erfindungsgemäßen Ausgestaltung nach Anspruch 1 weist die Hülle wenigstens einen ersten und einen zweiten Bereich auf, wobei der erste Bereiche zum proximalen Ende der Einführvorrichtung am Übergang zwischen Außenschaft und Aufnahmeelement angeordnet ist und geschrumpft ist und der zweite Bereich zum distalen Ende über dem Aufnahmeelement angeordnet ist und weniger geschrumpft ist als der erste Bereich. Die schwimmende Lagerung über dem Aufnahmeelementerlaubt eine besonders stützende Wirkung der Hülle am Übergang zwischen Außenschaft und Aufnahmeelement. Insbesondere ist eine ausreichende Beweglichkeit des Aufnahmeelements gewährleistet.

Gemäß einer erfindungsgemäßen Ausgestaltung nach Anspruch 9 weist die Hülle Schlitze auf. Damit kann die Flexibilität der Hülle, besonders im geschrumpften Zustand, beeinflusst werden. Vorteilhaft können die Schlitze am Übergang zwischen Außenschaft und Aufnahmeelement angeordnet sein, so dass dort eine hinreichende Flexibilität gewährleistet ist.

Gemäß einer günstigen Ausgestaltung kann das Aufnahmeelement zum Freisetzen eines selbstexpandierenden Stentsystems ausgebildet sein. Bei solchen Stentsystemen ist es besonders vorteilhaft, wenn eine Repositionierung des Stents möglich ist, indem ein sicheres Zurückführen des Stents in das Aufnahmeelement erfolgen kann.

Gemäß einer anderen vorteilhaften Ausgestaltung der Erfindung ist das Aufnahmeelement zum Freisetzen einer Herzklappenprothese ausgebildet ist, die über einen Katheter implantiert wird und ein selbstexpandierendes Grundgerüst aufweist, an dem die eigentlichen Herzklappen befestigt sind. Besonders bevorzugt ist die Herzklappenprothese zum Ersatz der natürlichen Aortenklappe ausgebildet.

Die Erfindung ist nachfolgend beispielhaft, anhand von in Zeichnungen dargestellten Ausführungsbeispielen, näher erläutert. Es zeigen in schematischer Darstellung:
- Fig. 1: eine Ansicht einer Einführvorrichtung nach einem Ausführungsbeispiel der Erfindung, in Form eines Katheters mit einer Kapsel als Aufnahmeelement für ein Implantat;
- Fig. 2: eine Detailansicht am distalen Ende der Einführvorrichtung aus Figur 1; und
- Fig. 3: ein Detail einer Hülle nach einem Ausführungsbeispiel der Erfindung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

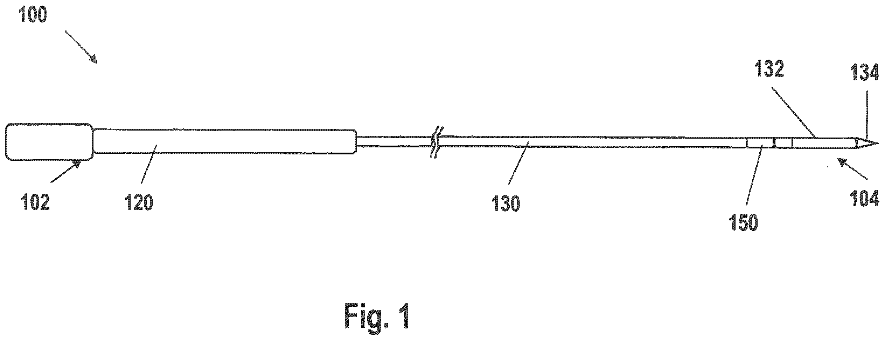

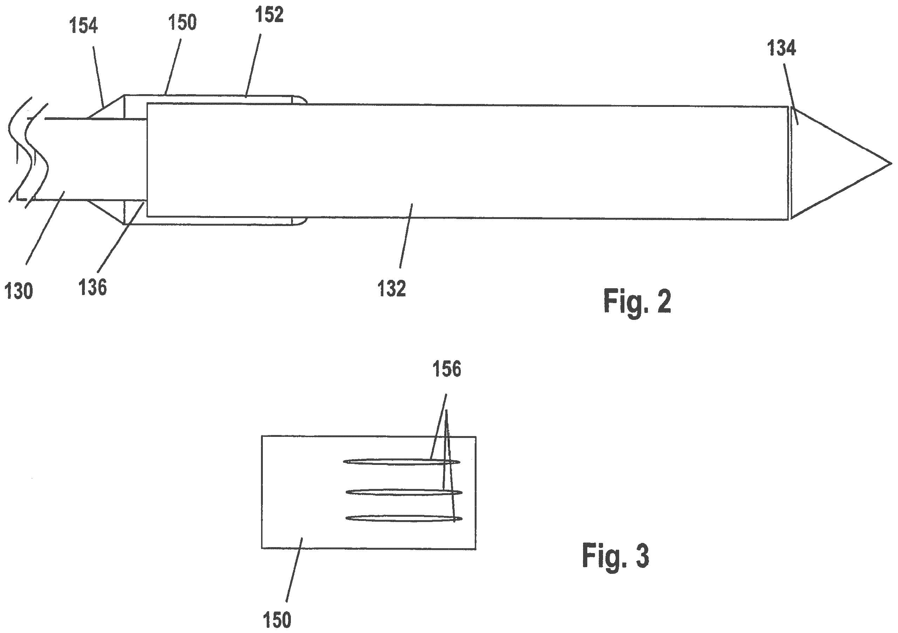

Figur 1 zeigt eine Ansicht einer Einführvorrichtung 100 nach einem Ausführungsbeispiel der Erfindung, in Form eines Katheters mit einem Außenschaft 130, und mit einer Kapsel als Aufnahmeelement 132 für ein Implantat. Figur 2 zeigt eine Detailansicht am distalen Ende der Einführvorrichtung 100 aus Figur 1.

Der Außenschaft 130 weist ein proximales Ende 102 mit einem Handgriff 120 und ein diesem gegenüberliegendes distales Ende 104 mit einer Spitze 134 auf. Zwischen proximalem und distalem Ende 102, 104 erstreckt sich ein Außenschaft 130 mit einem Aufnahmeelement 130, an das sich am distalen Ende 104 die Spitze 134 anschließt.

Die Einführvorrichtung 100 dient zum Einführen eines medizinischen Implantats (nicht dargestellt) in einen menschlichen und/oder tierischen Körper mit wenigstens einem Außenschaft 130. Das Implantat kann vorteilhaft ein selbstexpandierendes Stentsystem sein. Derartige Stentsysteme müssen besonders genau platziert werden, so dass die Einführvorrichtung 100 eine etwaige sichere Repositionierung gewährleisten soll. Das Implantat wird in einem mit dem Außenschaft 130 verbundenen Aufnahmeelement 132 zum Einsatzort transportiert und dort freigesetzt. Bei einer fehlerhaften Positionierung wird das am Einsatzort freigesetzte Implantat wieder in das Aufnahmeelement 132 zurückgezogen und an einer korrigierten Position wieder freigesetzt.

Der Außenschaft 130 bildet generell die radial äußere Begrenzung der Einführvorrichtung 100. Insbesondere können innerhalb des Außenschafts 130 ein oder mehrere Innenschäfte angeordnet sein, wie sie bei derartigen Einführsystemen üblich und dem Fachmann bekannt sind.

Der Außenschaft 130 und das Aufnahmeelement 132 sind an einem Übergang zwischen Außenschaft 130 und Aufnahmeelement 132 von einer lokal platzierten Hülle 150 umgeben, welche zumindest einen Teil der mechanischen Zug- und/oder Druckspannungen aufnimmt, die im gebogenen Zustand des Außenschaft 130 vorhanden sind. Würden diese Spannungen nicht gedämpft, könnte es zu einer Deformation bzw. zu einem Knick am Übergang zwischen Außenschaft 130 und Aufnahmeelement 132 führen, was die Funktion des Einführelements negativ beeinflussen kann.

Während der Außenschaft 130 üblicherweise eine beträchtliche Länge aufweist, beispielsweise mehr als einen Meter, ist die Hülle 150 kurz, beispielsweise wenige Zentimeter, insbesondere 3 cm. Typische Durchmesser des Außenschaft 130 und des Aufnahmeelements 132 sind wenige Millimeter, etwa 5 mm bis 6 mm. Die genauen Werte hängen von dem konkreten Design des Einführsystems ab und sind leicht anpassbar.

Die Hülle 150 überdeckt ein proximales Ende 136 des Aufnahmeelements 132 in einem ersten Bereich 152 und einen an das Aufnahmeelement 132 angrenzenden Bereich 154 des Außenschafts 130. Dabei ist es mechanisch vorteilhaft, wenn 25% bis 35% der Länge der Hülle 150 über dem Außenschaft 130 angeordnet sind und 75% bis 65% über dem Aufnahmeelement 132.

Die Hülle 150 ist über dem Außenschaft 130 geschrumpft, während sie über dem Aufnahmeelement 132 wenig oder gar nicht geschrumpft ist, so dass sie über dem Aufnahmeelement 132 schwimmend angeordnet ist. Dies gewährleistet die Beweglichkeit des Aufnahmeelements 132. In einem Ausführungsbeispiel ist eine Hülle 150 mit einer Länge von 3 cm und einem Innendurchmesser (ungeschrumpft) von 6,2 mm und einem Außendurchmesser von 6,4 mm eingesetzt. Nach dem Schrumpfen ist der Innendurchmesser über dem Außenschaft 130 in diesem Beispiel 5,9 mm und der Außendurchmesser 6,1 mm. Vorteilhaft ist der Übergangsbereich zwischen Außenschaft 130 und dem als Kapsel ausgebildeten Aufnahmeelements 132 gegen Abknicken geschützt.

### Vorteilhaft ist die Hülle 150 als Schrumpfschlauch aus PEEK gebildet

Figur 3 zeigt ein Detail einer Hülle 150 nach einem Ausführungsbeispiel der Erfindung. Die Hülle 150 ist mit in axialer Richtung ausgebildeten Schlitzen 156 versehen. Vorteilhaft sind die Schlitze 156 am Übergang zwischen Außenschaft 130 und Aufnahmeelement 132 angeordnet.

Insgesamt ermöglicht die erfindungsgemäße Einführvorrichtung 100 eine Vereinfachung des Freisetzprozesses zu einer genaueren und homogeneren Positionierung eines Implantats, sowie die Freigabe und das Zurückführen des Implantats.

Besonders günstig ist die erfindungsgemäße Einführvorrichtung 100 bei der Freisetzung von selbstexpandierenden Stentsystemen, zum Rückziehen des Schutzes von ballonexpandierenden Stents oder zum Rückziehen des Schutzes von beschichteten Ballonen. Das Design ist einfach zu realisieren und preiswert.

## Patentansprüche

1. Einführvorrichtung (100) zum Einführen eines medizinischen Implantats in einen menschlichen und/oder tierischen Körper mit einem Außenschaft (130), der ein proximales Ende (102) und ein diesem gegenüberliegendes distales Ende (104) aufweist, und einem am distalen Ende (104) mit dem Außenschaft (130) verbundenen Aufnahmeelement (132) für das Implantat, in dem das Implantat an seinen Bestimmungsort geführt wird, wobei das Aufnahmeelement eine Kapsel ist, wobei der Außenschaft (130) und das Aufnahmeelement (132) an einem Übergang zwischen Außenschaft (130) und Aufnahmeelement (132) von einer lokal platzierten Hülle (150) umgeben sind, welche mechanische Zug- und/oder Druckspannungen aufnimmt, wobei die Hülle (150) wenigstens einen ersten und einen zweiten Bereich (152, 154) aufweist, wobei der erste Bereich (152) am Übergang zwischen Außenschaft (130) und Aufnahmeelement (132) angeordnet ist und geschrumpft ist und der zweite Bereich (154) über dem Aufnahmeelement (132) angeordnet ist und weniger geschrumpft ist als der erste Bereich (152).

2. Einführvorrichtung nach Anspruch 1, **dadurch gekennzeichnet, dass** die Hülle (150) wenigstens ein proximales Ende (136) des Aufnahmeelements (132) überdeckt.

3. Einführvorrichtung nach Anspruch 1 oder 2, **dadurch gekennzeichnet, dass** die Hülle (150) aus einem Material gebildet ist, das gezielt von einem bezogen auf einen Innendurchmesser der Hülle (150) geweiteten Zustand in einen geschrumpften Zustand kontrahierbar ist.

4. Einführvorrichtung nach einem der vorhergegangenen Ansprüche, **dadurch gekennzeichnet, dass** die Hülle (150) Schlitze (156) aufweist.

5. Einführvorrichtung zumindest nach Anspruch 4, **dadurch gekennzeichnet, dass** die Schlitze (156) am Übergang zwischen Außenschaft (130) und Aufnahmeelement (132) angeordnet sind.

6. Einführvorrichtung nach einem der vorhergegangenen Ansprüche, **dadurch gekennzeichnet, dass** die Hülle (150) aus PEEK gebildet ist.

7. Einführvorrichtung nach einem der vorhergegangenen Ansprüche, **dadurch gekennzeichnet, dass** das Aufnahmeelement (132) zum Freisetzen und/oder Zurückziehen eines selbstexpandierenden Stentsystems ausgebildet ist.

8. Einführvorrichtung nach einem der vorhergegangenen Ansprüche, **dadurch gekennzeichnet, dass** das Aufnahmeelement (132) zum Freisetzen und/oder Zurückziehen eines Ballonsystems ausgebildet ist.

9. Einführvorrichtung (100) zum Einführen eines medizinischen Implantats in einen menschlichen und/oder tierischen Körper mit einem Außenschaft (130), der ein proximales Ende (102) und ein diesem gegenüberliegendes distales Ende (104) aufweist, und einem am distalen Ende (104) mit dem Außenschaft (130) verbundenen Aufnahmeelement (132) für das Implantat, in dem das Implantat an seinen Bestimmungsort geführt wird, wobei der Außenschaft (130) und das Aufnahmeelement (132) an einem Übergang zwischen Außenschaft (130) und Aufnahmeelement (132) von einer lokal platzierten Hülle (150) umgeben sind, welche mechanische Zug- und/oder Druckspannungen aufnimmt, wobei die Hülle (150) Schlitze (156) aufweist.

10. Einführvorrichtung nach Anspruch 9, **dadurch gekennzeichnet, dass** die Hülle (150) wenigstens ein proximales Ende (136) des Aufnahmeelements (132) überdeckt.

11. Einführvorrichtung nach Anspruch 9 oder 10, **dadurch gekennzeichnet, dass** die Hülle (150) aus einem Material gebildet ist, das gezielt von einem bezogen auf einen Innendurchmesser der Hülle (150) geweiteten Zustand in einen geschrumpften Zustand kontrahierbar ist.

12. Einführvorrichtung nach einem der Ansprüche 9 bis 11, **dadurch gekennzeichnet, dass** die Hülle (150) wenigstens einen ersten und einen zweiten Bereich (152, 154) aufweist, wobei der erste Bereich (152) am Übergang zwischen Außenschaft (130) und Aufnahmeelement (132) angeordnet ist und geschrumpft ist und der zweite Bereich (154) über dem Aufnahmeelement (132) angeordnet ist und weniger geschrumpft ist als der erste Bereich (152).

13. Einführvorrichtung zumindest nach Anspruch 9, **dadurch gekennzeichnet, dass** die Schlitze (156) am Übergang zwischen Außenschaft (130) und Aufnahmeelement (132) angeordnet sind.

14. Einführvorrichtung nach einem der Ansprüche 9 bis 13, **dadurch gekennzeichnet, dass** die Hülle (150) aus PEEK gebildet ist.

15. Einführvorrichtung nach einem der Ansprüche 9 bis 14, **dadurch gekennzeichnet, dass** das Aufnahmeelement (132) zum Freisetzen und/oder Zurückziehen eines selbstexpandierenden Stentsvstems ausgebildet ist.

16. Einführvorrichtung nach einem der Ansprüche 9 bis 15, **dadurch gekennzeichnet, dass** das Aufnahmeelement (132) zum Freisetzen und/oder Zurückziehen eines Ballonsystems ausgebildet ist.

17. Einführvorrichtung (100) zum Einführen eines medizinischen Implantats in einen menschlichen und/oder tierischen Körper mit einem Außenschaft (130), der ein proximales Ende (102) und ein diesem gegenüberliegendes distales Ende (104) aufweist, und einem am distalen Ende (104) mit dem Außenschaft (130) verbundenen Aufnahmeelement (132) für das Implantat, in dem das Implantat an seinen Bestimmungsort geführt wird, wobei der Außenschaft (130) und das Aufnahmeelement (132) an einem Übergang zwischen Außenschaft (130) und Aufnahmeelement (132) von einer lokal platzierten Hülle (150) umgeben sind, welche mechanische Zug- und/oder Druckspannungen aufnimmt, wobei die Hülle (150) über dem Außenschaft (130) geschrumpft ist und über dem Aufnahmeelement (132) wenig oder gar nicht geschrumpft ist, so dass sie über dem Aufnahmeelement (132) schwimmend angeordnet ist.

18. Einführvorrichtung nach Anspruch 17, **dadurch gekennzeichnet, dass** die Hülle (150) aus einem Material gebildet ist, das gezielt von einem bezogen auf einen Innendurchmesser der Hülle (150) geweiteten Zustand in einen geschrumpften Zustand kontrahierbar ist.

19. Einführvorrichtung nach einem der Ansprüche 17 bis 18, **dadurch gekennzeichnet, dass** die Hülle (150) aus PEEK gebildet ist.

20. Einführvorrichtung nach einem der Ansprüche 17 bis 19. **dadurch gekennzeichnet, dass** das Aufnahmeelement (132) zum Freisetzen und/oder Zurückziehen eines selbstexpandierenden Stentsystems ausgebildet ist.

## Claims

1. An insertion device (100) for the insertion of a medical implant into a human and/or animal body, the insertion device comprising an outer shaft (130), which has a proximal end (102) and a distal end (104) opposite said proximal end, and a receiving element (132) for the implant, which receiving element is connected at the distal end (104) to the outer shaft (130) and in which receiving element the implant is guided to its intended location, wherein the receiving element is a capsule, wherein the outer shaft (130) and the receiving element (132) are surrounded, at a transition between outer shaft (130) and receiving element (132), by a locally placed sleeve (150) which takes up mechanical tensile and/or compressive stresses, wherein the sleeve (150) has at least a first region and a second region (152, 154), wherein the first region (152) is arranged at the transition between outer shaft (130) and receiving element (132) and is shrunk, and the second region (154) is arranged above the receiving element (132) and is shrunk to a lesser extent than the first region (152).

2. The insertion device according to claim 1, **characterised in that** the sleeve (150) covers at least a proximal end (136) of the receiving element (132).

3. The insertion device according to claim 1 or 2, **characterised in that** the sleeve (150) is formed from a material that can be contracted selectively from a state that is widened based on an inner diameter of the sleeve (150) into a shrunken state.

4. The insertion device according to one of the preceding claims, **characterised in that** the sleeve (150) comprises slits (156).

5. The insertion device at least according to claim 4, **characterised in that** the slits (156) are arranged at the transition between outer shaft (130) and receiving element (132).

6. The insertion device according to one of the preceding claims, **characterised in that** the sleeve (150) is formed from PEEK.

7. The insertion device according to one of the preceding claims, **characterised in that** the receiving element (132) is designed for the release and/or withdrawal of a self-expanding stent system

8. The insertion device according to one of the preceding claims, **characterised in that** the receiving element (132) is designed for the release and/or withdrawal of a balloon system.

9. An insertion device (100) for the insertion of a medical implant into a human and/or animal body, the insertion device comprising an outer shaft (130), which has a proximal end (102) and a distal end (104) opposite said proximal end, and a receiving element (132) for the implant, which receiving element is connected at the distal end (104) to the outer shaft (130) and in which receiving element the implant is guided to its intended location, wherein the outer shaft (130) and the receiving element (132) are surrounded, at a transition between outer shaft (130) and receiving element (132), by a locally placed sleeve (150), which takes up mechanical tensile and/or compressive stresses, wherein the sleeve (150) comprises slits (156).

10. The insertion device according to claim 9, **characterised in that** the sleeve (150) covers at least a proximal end (136) of the receiving element (132).

11. The insertion device according to claim 9 or 10, **characterised in that** the sleeve (150) is formed from a material that can be contracted selectively from a state that is widened based on an inner diameter of the sleeve (150) into a shrunken state.

12. The insertion device according to one of claims 9 to 11, **characterised in that** the sleeve (150) has at least a first region and a second region (152, 154), wherein the first region (152) is arranged at the transition between outer shaft (130) and receiving element (132) and is shrunk, and the second region (154) is arranged above the receiving element (132) and is shrunk to a lesser extent than the first region (152).

13. The insertion device at least according to claim 9, **characterised in that** the slits (156) are arranged at the transition between outer shaft (130) and receiving element (132).

14. The insertion device according to one of claims 9 to 13, **characterised in that** the sleeve (150) is formed from PEEK.

15. The insertion device according to one of claims 9 to 14, **characterised in that** the receiving element (132) is designed for the release and/or withdrawal of a self-expanding stent system.

16. The insertion device according to one of claims 9 to 15, **characterised in that** the receiving element (132) is designed for the release and/or withdrawal of a balloon system.

17. An insertion device (100) for the insertion of a medical implant into a human and/or animal body, the insertion device comprising an outer shaft (130), which has a proximal end (102) and a distal end (104) opposite said proximal end, and a receiving element (132) for the implant, which receiving element is connected at the distal end (104) to the outer shaft (130) and in which receiving element the implant is guided to its intended location, wherein the outer shaft (130) and the receiving element (132) are surrounded at a transition between outer shaft (130) and receiving element (132) by a locally placed sleeve (150), which takes up mechanical tensile and/or compressive stresses, wherein the sleeve (150) is shrunk over the outer shaft (130) and is shrunk to a lesser extent or is not shrunk over the receiving element (132), such that the sleeve is arranged in a floating manner over the receiving element (132).

18. The insertion device according to claim 17, **characterised in that** the sleeve (150) is formed from a material that can be contracted selectively from a state that is widened based on an inner diameter of the sleeve (150) into a shrunken state.

19. The insertion device according to one of claims 17 to 18, **characterised in that** the sleeve (150) is formed from PEEK.

20. The insertion device according to one of claims 17 to 19, **characterised in that** the receiving element (132) is designed for the release and/or withdrawal of a self-expanding stent system.

## Revendications

1. Dispositif d'insertion (100) permettant d'insérer un implant médical dans un corps humain et/ou animal, doté d'une tige extérieure (130), qui présente une extrémité proximale (102) et une extrémité distale (104) lui étant opposée, et d'un élément de réception (132) pour l'implant, relié avec la tige extérieure (130) à l'extrémité distale (104), dans lequel l'implant est mené jusqu'à son emplacement de destination, où l'élément de réception est une capsule, où la tige extérieure (130) et l'élément de réception (132) sont entourés par une enveloppe (150), placée localement au niveau d'une transition entre la tige extérieure (130) et l'élément de réception (132), laquelle enveloppe absorbe des tensions mécaniques de traction et/ou de compression, où l'enveloppe (150) présente au moins une première et une deuxième zone (152, 154), où la première zone (152) est disposée au niveau de la transition entre la tige extérieure (130) et l'élément de réception (132) et est rétrécie et la deuxième zone (154) est disposée au-dessus de l'élément de réception (132) et est moins rétrécie que la première zone (152).

2. Dispositif d'insertion selon la revendication 1, **caractérisé en ce que** l'enveloppe (150) recouvre au moins une extrémité proximale (136) de l'élément de réception (132).

3. Dispositif d'insertion selon la revendication 1 ou la revendication 2, **caractérisé en ce que** l'enveloppe (150) est formée à base d'un matériau qui peut se contacter de manière ciblée d'un état élargi par rapport à un diamètre intérieur de l'enveloppe (150) vers un état rétréci.

4. Dispositif d'insertion selon l'une des revendications précédentes, **caractérisé en ce que** l'enveloppe (150) présente des fentes (156).

5. Dispositif d'insertion selon la revendication 4, **caractérisé en ce que** les fentes (150) sont disposées au niveau de la transition entre la tige extérieure (130) et l'élément de réception (132).

6. Dispositif d'insertion selon l'une des revendications précédentes, **caractérisé en ce que** l'enveloppe (150) est en PEEK.

7. Dispositif d'insertion selon l'une des revendications précédentes, **caractérisé en ce que** l'élément de réception (132) est conçu pour libérer et/ou retirer un système de stent auto-expansible.

8. Dispositif d'insertion selon l'une des revendications précédentes, **caractérisé en ce que** l'élément de réception (132) est conçu pour la libération et/ou le retrait d'un système de ballonnet.

9. Dispositif d'insertion (100) permettant d'insérer un implant médical dans un corps humain et/ou animal, doté d'une tige extérieure (130), qui présente une extrémité proximale (102) et une extrémité distale (104) lui étant opposée, et d'un élément de réception (132) pour l'implant relié avec la tige extérieure (130) à l'extrémité distale (104), dans lequel l'implant est mené jusqu'à son emplacement de destination, où la tige extérieure (130) et l'élément de réception (132) sont entourés par une enveloppe (150), placée localement au niveau d'une transition entre la tige extérieure (130) et l'élément de réception (132), laquelle enveloppe absorbe des tensions mécaniques de traction et/ou de compression, où l'enveloppe (150) présente des fentes (156).

10. Dispositif d'insertion selon la revendication 9, **caractérisé en ce que** l'enveloppe (150) recouvre au moins une extrémité proximale (136) de l'élément de réception (132).

11. Dispositif d'insertion selon la revendication 9 ou la revendication 10, **caractérisé en ce que** l'enveloppe (150) est formée dans un matériau qui peut se contacter de manière ciblée d'un état élargi par rapport à un diamètre intérieur de l'enveloppe (150) vers un état rétréci.

12. Dispositif d'insertion selon l'une des revendications 9 à 11, **caractérisé en ce que** l'enveloppe (150) présente au moins une première et une deuxième zone (152, 154), où la première zone (152) est disposée au niveau de la transition entre la tige extérieure (130) et l'élément de réception (132) et est rétrécie et la deuxième zone (154) est disposée au-dessus de l'élément de réception (132) et est moins rétrécie que la première zone (152).

13. Dispositif d'insertion selon la revendication 9, **caractérisé en ce que** les fentes (150) sont disposées au niveau de la transition entre la tige extérieure (130) et l'élément de réception (132).

14. Dispositif d'insertion selon l'une des revendications 9 à 13, **caractérisé en ce que** l'enveloppe (150) est en PEEK.

15. Dispositif d'insertion selon l'une des revendications 9 à 14, **caractérisé en ce que** l'élément de réception (132) est conçu pour la libération et/ou le retrait d'un système de stent auto-expansible.

16. Dispositif d'insertion selon l'une des revendications 9 à 15, **caractérisé en ce que** l'élément de réception (132) est conçu pour la libération et/ou le retrait d'un système de ballonnet.

17. Dispositif d'insertion (100) permettant d'insérer un implant médical dans un corps humain et/ou animal, doté d'une tige extérieure (130), qui présente une extrémité proximale (102) et une extrémité distale (104) lui étant opposée, et d'un élément de réception (132) pour l'implant relié avec la tige extérieure (130) à l'extrémité distale (104), dans lequel l'implant est mené jusqu'à son emplacement de destination, où la tige extérieure (130) et l'élément de réception (132) sont entourés par une enveloppe (150), placée localement au niveau d'une transition entre la tige extérieure (130) et l'élément de réception (132), laquelle enveloppe absorbe des tensions mécaniques de traction et/ou de compression, où l'enveloppe (150) est retroussée au-dessus de la tige extérieure (130) et n'est que peu ou pas du tout retroussée au-dessus de l'élément de réception (132), de sorte qu'elle est disposée librement par-dessus l'élément de réception (132).

18. Dispositif d'insertion selon la revendication 17, **caractérisée en ce que** l'enveloppe (150) est formée à base d'un matériau qui peut se contacter de manière ciblée d'un état élargi par rapport à un diamètre intérieur de l'enveloppe (150) vers un état rétréci.

19. Dispositif d'insertion selon l'une des revendications 17 à 18, **caractérisé en ce que** l'enveloppe (150) est en PEEK.

20. Dispositif d'insertion selon l'une des revendications 17 à 19, **caractérisé en ce que** l'élément de réception (132) est conçu pour la libération et/ou le retrait d'un système de stent auto-expansible.
